(19) Europäisches Patentamt
European Patent Office
Office européen des brevets

(11) **EP 4 764 486 A1**

(12) **EUROPÄISCHE PATENTANMELDUNG**

(43) Veröffentlichungstag:
**24.06.2026 Patentblatt 2026/26**

(21) Anmeldenummer: 24220940.1

(22) Anmeldetag: **18.12.2024**

(51) Internationale Patentklassifikation (IPC):
**G01N 27/90** $^{(2021.01)}$

(52) Gemeinsame Patentklassifikation (CPC):
**G01N 27/9006;** G01N 33/20

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Primetals Technologies Germany GmbH**
**91058 Erlangen (DE)**

(72) Erfinder:
• **Champion, Nicholas John**
**4210 Gallneukirchen (AT)**

• **Hirschmanner, Martin**
**4060 Leonding (AT)**
• **Kirchschlager, Raimund**
**4040 Linz (AT)**
• **Koppler, Alois**
**4020 Linz (AT)**
• **Lang, Oliver**
**4407 Dietach (AT)**

(74) Vertreter: **Metals@Linz**
**Primetals Technologies Austria GmbH**
**Intellectual Property Upstream IP UP**
**Turmstraße 44**
**4031 Linz (AT)**

(54) **VORRICHTUNG UND VERFAHREN ZUR BERÜHRUNGSLOSEN ERMITTLUNG VON MATERIALEIGENSCHAFTEN MAGNETISIERBARER METALLBÄNDER UND BLECHE**

(57) Das berührungslose Ermitteln mechanischer und/oder elektromagnetischer Materialeigenschaften $Q_i$ eines magnetisierbaren metallischen Flachprodukts 2 erfolgt mittels einer Vorrichtung 1, die eine Spulenanordnung 4, eine regelbare Stromquelle 10 und eine Steuer- und Messeinheit 12 umfasst. Die Spulenanordnung 4 weist zumindest eine erste Spuleneinheit 41 mit einer Erregerspule 51, einem magnetisierbaren Joch 6 und Messspulen 81, 82 auf. Das Joch 6, das von der Erregerspule 51 umschlossen wird, ist U-förmig ausgebildet. In zyklischen Messdurchläufen $M_k$ wird die Erregerspule 51 sequentiell mit Wechselstrom einer oder mehrerer Grundfrequenzen $f_j$ zwischen 10Hz und 1000Hz beaufschlagt, wobei die Steuer- und Messeinheit 12 in den Messspulen 81, 82 ein zu der jeweiligen Grundfrequenz $f_j$ korrespondierendes Antwortsignal $r_j$ detektiert.

FIG 2

**Beschreibung**

Die Erfindung betrifft eine Vorrichtung und ein Verfahren zum berührungslosen Ermitteln mechanischer und/oder elektromagnetischer Materialeigenschaften von magnetisierbaren metallischen Flachprodukten in Prozesslinien oder Walz- und Stanzanlagen.

Die probenbasierte Messung von Materialeigenschaften von magnetisierbaren Stahlbändern oder Blechen, die im Rahmen der Erfindung einfach als 'Flachprodukte' bezeichnet werden - ist in der Regel zerstörend, stichprobenartig (üblicherweise werden die Proben lediglich in der Nähe eines Bandanfanges oder Blechrandes genommen) und aufwendig - beispielsweise mittels Zugfestigkeitsbeprobung - möglich. Alternativ dazu sind zwar zerstörungsfrei, auf induktiver Basis arbeitende Messgeräte bekannt, die jedoch verschiedene Nachteile aufweisen.

Aus der DE 10 2019 109 337 B3 ist eine Vorrichtung bekannt, die zur Vermessung eines Metallbandes eine Erregerspule zur Induktion eines magnetischen Flusses in dem Metallband, eine Sekundärspule zur Messung des gesamten magnetischen Flusses sowie jeweils ein oder mehrere Sondenspulen und dazu korrespondierende Feldspulen umfasst. Nachteilig ist der große bauliche Aufwand für die das Metallband umgreifende Erreger- bzw. Sekundärspule, die dieses in seiner möglichen Maximalbreite begrenzen. Zudem sind die Feldspulen herstellungsaufwendig, da sie den induzierten magnetischen Fluss in einer Ebene parallel zur Oberfläche des Metallbandes erfassen und daher entsprechend flach ausgestaltet sein müssen. Weiterhin können mit der offenbarten Vorrichtung nur magnetische Eigenschaften in Bandlängsrichtung eines Metallbandes, jedoch keine mechanischen Eigenschaften gemessen werden.

Aus der Vortragsschrift 'Online Quality Monitoring of IF and High Strength Steels on Continuous Galvanizing Lines Controlled by Furnace Mathematical Model' der Messe METEC & 2nd ESTAD 2015 vom 15.-19. Juni 2015 in Düsseldorf oder aus dem Artikel 'Materialcharakterisierung an Flachprodukten verbessert die Prozesskontrolle' aus "stahl und eisen" 132 (2012), Heft 07, Seiten 80-90, ist das sogenannte IMPOC-Verfahren bekannt, das auf einer lokalen Aufmagnetisierung eines zu vermessenden Stahlbandes mit Hilfe einer mit gepulstem Stromfluss angespeisten Induktionsspule basiert. Dabei wird von einer Messspule das Streufeld des magnetisierten Bandes erfasst und daraus werden mittels einer multiplen linearen Regressionsrechnung, die zusätzlich weitere Prozessparameter für das Band erfordert, die Zugfestigkeit und die Streckgrenze als mechanische Eigenschaften des Bandes ermittelt. Nachteilig an diesem Verfahren ist der Umstand, dass für eine Messung an einem Metallband, welches beispielsweise in einer Prozesslinie einer stahlverarbeitenden Anlage geführt wird, die Messung nicht in beliebiger Richtung, sondern nur nur in Bewegungsrichtung des Bandes erfolgen kann und dafür eine bestimmte Mindestgeschwindigkeit des Bandes relativ zu dem Messkopf erforderlich ist.

Weiterhin ist das sogenannte HACOM-Verfahren beispielsweise aus der EP 0 833 150 B1, der US 5,144,565 A und dem vorgenannten Artikel 'Materialcharakterisierung an Flachprodukten verbessert die Prozesskontrolle' aus "stahl und eisen" 132 (2012), Heft 07, Seiten 80-90, bekannt, bei dem über ein Spulensystem einem Prüfobjekt (in der Regel einem magnetisierbaren Metallband) ein sinsusförmig modulierter magnetischer Fluss, der auch als 'Grundwelle' bezeichnet wird, aufgeprägt wird. Die durch die Grundwelle hervorgerufene Magnetisierung des Prüfobjekts durchläuft eine materialspezifische Hysteresekurve und erzeugt zusammen mit den ebenfalls in dem Prüfobjekt von der Grundwelle hervorgerufenen Wirbelströmen ein zur Grundwelle korrespondierendes Sekundärfeld, dessen genaue Ausprägung von den spezifischen Materialeigenschaften des Prüfobjekts abhängt. Das Sekundärfeld wird von einer Empfängerspule detektiert und in der Folge in höherharmonische Anteile in Bezug auf die Grundwelle zerlegt. Daraus kann wiederum mittels ein- oder mehrdimensionaler Regressionsanalyse auf mechanische Eigenschaften wie z.B. die Zugfestigkeit und die Dehngrenze des Prüfobjekts geschlossen werden.

Nachteilig an den geoffenbarten HACOM-Vorrichtungen und den korrespondierenden Verfahren ist der Umstand, dass zur Erzeugung der Grundwelle lediglich Luftspulen ohne Magnetjoch verwendet werden, sodass nur ein relativ geringer magnetischer Fluss im Prüfobjekt induziert werden kann. Dies ist dem Umstand geschuldet, dass jedes herkömmliche Magnetjoch (z.B. aus lamelliertem Elektroblech) bei Anlegen eines äußeren periodischen Feldes seinerseits Ummagnetisierungsverlusten unterworfen ist und Wirbelströme im Magnetjoch entstehen. Daher würde in dem Magnetjoch selbst ein entsprechendes Gegenfeld mit höherharmonischen Anteilen generiert werden, sodass zwischen dem Gegenfeld des Prüfobjekts und jenem des Magnetjochs nicht unterschieden werden könnte und eine zuverlässige Auswertung des Prüfobjets nicht möglich wäre.

Aufgrund der genannten Umstände müssen die Luftspulen bei den vorgenannten HACOM-Vorrichtungen und -Verfahren in nachteiliger Weise in einem sehr geringen Abstand zu dem Prüfobjekt beabstandet werden; zudem ist aufgrund des geringen induzierten magnetischen Flusses eine genaue Vermessung von einigen Materialklassen nicht mit ausreichender Genauigkeit möglich. Weiterhin erfordern Luftspulen Kompensationsspulen sowie ein relativ großes umgebendes Volumen, das frei von metallischen Körpern ist, um unerwünschte Gegenfelder zu vermeiden. Dies wirkt sich nachteilig auf die Einbaumöglichkeit solcher Sensoren an einer metallverarbeitenden Anlage aus.

Zudem ist an den bislang bekannten HACOM-Verfahren nachteilig, dass magnetische Eigenschaften eines Prüfobjekts nicht mit ausreichender Genauigkeit ermittelt werden können, was der geringen, im Prüfobjekt mittels der Luftspulen induzierbaren magnetischen Feldstärke geschuldet ist.

Die vorgenannte Publikation 'Materialcharakterisierung an Flachprodukten verbessert die Prozesskontrolle' in "stahl und eisen" 132 (2012), Heft 07, Seiten 80-90, beschreibt auch das sogenannte MFIA-Verfahren, bei dem einem Metallband über Induktionsspulen auf einem H-förmigen Ferritjoch ein magnetisches Wechselfeld aufgeprägt wird. Mittels Messpulen wird das Gegenfeld des Bandes erfasst und ausgewertet, woraus auf materialspezifische Eigenschaften des Bandmaterials geschlossen werden kann. Nachteilig an einem derartigen Messaufbau ist der Umstand, dass an der bandabgewandten Seite des Jochs zusätzliche, sogenannte 'Kompensationsspulen' erforderlich sind: einerseits wird dadurch die Bauhöhe der Messanordnung vergrößert, andererseits wird das in den Messpulen erzeugte Signal von jeglichen metallischen Objekten in der näheren Umgebung (insbesondere auch von dem zu vermessenden Metallband selbst) beeinflusst und verfälscht, weil die erzeugten Feldlinien der magnetischen Flussdichte B (im Folgenden einfach als 'magnetische Feldlinien' bezeichnet) aufgrund der H-Form des Jochs nicht überwiegend innerhalb von diesem bzw. dem Metallband, sondern auch im umliegenden Außenraum geschlossen werden.

Es ist daher Aufgabe der Erfindung, die Nachteile der aus dem Stand der Technik bekannten Lösungen zu überwinden und eine verbesserte Vorrichtung und ein verbessertes Verfahren zur berührungslosen Ermittlung mechanischer bzw. elektromagnetischer Materialeigenschaften von magnetisierbaren Metallbändern bzw. Blechen anzugeben.

Die Aufgabe wird erfindungsgemäß durch eine Vorrichtung mit den Merkmalen des Anspruchs 1 und einem Verfahren mit den Merkmalen des Anspruchs 9 gelöst. Vorteilhafte Ausgestaltungen der Erfindung sind Gegenstand der Unteransprüche.

Zwischen einer statischen magnetischen Feldstärke H in einem bestimmten Material und der dadurch in dem Material induzierten magnetischen Flussdichte B besteht der Zusammenhang

$$B = \mu \cdot H \qquad\qquad (1)$$

wobei $\mu$ die magnetische Permeabilität des betreffenden Materials ist. Die physikalischen Einheiten dieser Größen werden im SI-System folgendermaßen angegeben:

$$[B] = T \quad (Tesla)$$
$$[H] = A/m \ (Ampere\ pro\ Meter)$$
$$[\mu] = H/m \ (Henry\ pro\ Meter) \qquad\qquad (2)$$

Die magnetische Permeabilität $\mu$ kann gemäß

$$\mu = \mu_0 \cdot \mu_r \qquad\qquad (3)$$

auch als Produkt zwischen der magnetischen Feldkonstante $\mu_0$ und der dimensionslosen, relativen Permeabilität $\mu_r$ des jeweiligen Materials ausgedrückt werden, wobei der Wert von $\mu_0$ näherungsweise $1.26 \cdot 10^{-6}$ H/m beträgt.

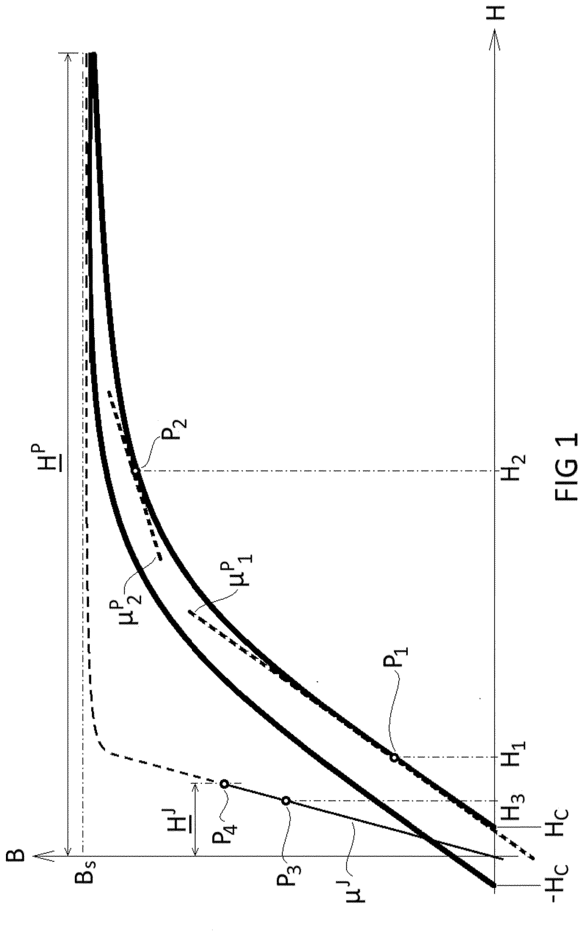

Bei Anliegen eines zeitvariablen magnetischen Wechselfeldes in einem Material kann der Zusammenhang zwischen magnetischer Flussdichte B und magnetischer Feldstärke H in Form eines Hysteresediagramms entsprechend FIG 1 dargestellt werden, wobei der Zusammenhang aus Formel (1) durch

$$\mu = \partial B / \partial H \qquad\qquad (4)$$

ersetzt wird. Anders ausgedrückt: im Fall von Wechselfeldern ist die magnetische Permeabilität $\mu$ durch die erste Ableitung der magnetischen Flussdichte B nach der magnetischen Feldstärke H bestimmt.

Bei Einbringen eines magnetisches Wechselfeldes einer bestimmten Grundfrequenz $f_j$ (der Index j bezeichnet hierbei eine bestimmte Grundfrequenz mit einem bestimmten Wert) in ein Material wird in diesem ein korrespondierendes Gegenfeld hervorgerufen, welches in Form eines Antwortsignals detektiert und zur Bestimmung spezifischer Eigenschaften des betreffenden Materials herangezogen werden kann. Die Genauigkeit der Bestimmung hängt dabei wesentlich vom Auftreten höherharmonischer Anteile (Anteile, deren Frequenz einen ganzzahlig vielfachen Wert der Grundfrequenz $f_j$ entspricht) im Antwortsignal ab. Derartige höherharmonische Anteile entstehen jedoch nur in Bereichen, in denen die Hysteresekurve des betreffenden Materials einen nichtlinearen Verlauf aufweist, weswegen zur Erzeugung von höherharmonischen Anteilen die magnetische Flussdichte B in dem zu untersuchenden Material in der Nähe der Sättigungsflussdichte $B_s$ liegen muss. Aufgrund der Punktsymmetrie von Hysteresekurven im Allgemeinen treten zudem nur ungeradzahlige höherharmonische Anteile im Antwortsignal auf.

Der zentrale Gedanke der vorliegenden Erfindung beruht darauf, einerseits mittels einer Erregerspule in einem zu untersuchenden Probenmaterial eine ausreichend hohe magnetische Flussdichte B nahe der Sättigungsflussdichte $B_s$ zu erzeugen, bei der der Zusammenhang mit der magnetischen Feldstärke H deutlich nichtlinear wird. Dies wird durch die Verwendung eines U-förmigen magnetisierbaren Jochs erreicht, das von der Erregerspule umschlossen wird und dessen Enden unter Ausbildung zweier Luftspalte einem flachen Probenmaterial zugewandt sind.

Andererseits darf die von der Erregerspule in dem Joch selbst erzeugte magnetische Feldstärke $H^J$ nur solche Werte annehmen, bei denen der Zusammenhang mit der magnetischen Flussdichte B im Joch (entsprechend der Hysteresekurve des Jochmaterials) noch in guter Näherung linear ist, da ansonsten in dem Joch selbst höherharmonische Anteile entstünden, die sich mit den höherharmonischen Anteilen des Probenmaterials überlagern würden, sodass in der Folge eine Auswertung von dessen Antwortsignal verschlechtert bzw. unmöglich würde.

Erfindungsgemäß wird daher eine relativ kurze Luftspaltstrecke zwischen dem Joch einer Erregerspule und einem zu vermessenden Material geschaffen, sodass ein von der Erregerspule hervorgerufener magnetischer Fluss $\Phi$ im Wesentlichen von dem magnetisierbaren Probenmaterial geschlossen wird. Anders ausgedrückt laufen dadurch - bildlich gesprochen - so gut wie keine aus dem Joch austretenden magnetischen Feldlinien am Probenmaterial vorbei sondern treten an den Luftspalten nahezu vollständig in das Probenmaterial ein bzw. aus diesem aus.

Zwischen einem magnetischen Fluss $\Phi$, der ein Material mit magnetischem Widerstand $R_m$ durchdringt, und einer assoziierten magnetischen Spannung $V_m$ gilt allgemein der Zusammenhang

$$\Phi \;=\; V_m/R_m \qquad\qquad\qquad (5)$$

Bei räumlich homogenem magnetischem Fluss $\Phi$ über einen Abschnitt der Länge l durch das Material (bildlich gesprochen verlaufen dabei die magnetische Feldlinien entlang des Abschnitts l im Wesentlichen mit konstanter Dichte und parallel zueinander) lassen sich der magnetische Widerstand $R_m$ gemäß

$$R_m \;=\; l\,/\,(\mu\cdot A) \qquad\qquad\qquad (6)$$

und die magnetische Spannung $V_m$ gemäß

$$V_m \;=\; H\cdot l \qquad\qquad\qquad (7)$$

ausdrücken, wobei $\mu$ die magnetische Permeabilität und A die von dem magnetischen Fluss $\Phi$ durchdrungene Querschnittsfläche des betreffenden Materials ist. Der magnetische Widerstand $R_m$ hängt allgemein von den Materialeigenschaften und von Form und Größe des betrachteten Materialabschnitts ab.

Wenn, wie voranstehend dargelegt, der von einer Erregerspule erzeugte magnetische Fluss $\Phi$ im Wesentlichen zur Gänze von dem Probenmaterial geschlossen wird und dabei sowohl im magnetisierbaren Joch als auch im Probenmaterial größtenteils homogen verläuft, dann lässt sich anhand von Formeln (5) bis (7) das Verhältnis der magnetischen Feldstärke $H^P$ im Probenmaterial zur magnetischen Feldstärke $H^J$ im Joch gemäß

$$H^P/H^J \;\propto\; (\mu^J/\mu^P)\cdot(A^J/A^P) \qquad\qquad\qquad (8)$$

abschätzen, wobei $\mu^J$ bzw. $\mu^P\cdot$die magnetische Permeabilität des Jochmaterials bzw. des Probenmaterials ist, wobei $A^J$ die Querschnittsfläche des Jochs bezeichnet und wobei $A^P$ eine effektive Querschnittsfläche des Probenmaterials darstellt, durch die ein Großteil des magnetische Flusses $\Phi$ innerhalb des Probenmaterials hindurchtritt.

Die exakte Ermittlung einer derartigen effektiven Querschnittsfläche $A^P$ innerhalb des Probenmaterials, die Bemaßung des Luftspaltes, um die voranstehend genannte Voraussetzung zu schaffen, dass der magnetische Fluss $\Phi$ im Wesentlichen zur Gänze von dem Probenmaterial geschlossen wird, sowie die Bemaßung des magnetisierbaren Jochs, um einen vorgenannten, auch im Probenmaterial größtenteils homogen verlaufenden magnetischen Fluss $\Phi$ zu erzielen, sind dem Fachmann bekannt und nicht Gegenstand der Erfindung. Beispielsweise kann eine entsprechende magnetische Feldkonfiguration durch Lösung der zugrundeliegenden physikalischen Differentialgleichungen mittels numerischer Verfahren ermittelt werden.

Zusammengefasst beruht der Kerngedanke der Erfindung auf der Überlegung, durch Verwendung eines magnetisierbaren Jochs aus einem Material mit geeigneter (insbesonders sehr hoher) magnetischer Permeabilität $\mu^J$ und entsprechender Formgebung bzw. Beabstandung zum Probenmaterial (zwecks Erzielung eines günstigen Verhältnisse der Querschnittsflächen gemäß Gleichung (8)) die magnetische Flussdichte B eines magnetischen Wechselfeldes mit der Grundfrequenz $f_j$ im Joch und in einem zu vermessenden Material so einzustellen, dass höherharmonische Anteile zur Grundfrequenz $f_j$ nur im Probenmaterial, jedoch nicht im magnetisierbaren Joch selbst entstehen.

Basierend auf den voranstehenden Ausführungen umfasst eine erfindungsgemäße Vorrichtung zum Ermitteln einer oder mehrerer Materialeigenschaften $Q_i$ eines Flachprodukts eine Spulenanordnung, eine - in Bezug auf eine vorzugebende Stromstärke - regelbare Stromquelle und eine Steuer- und Messeinheit. Die Spulenanordnung weist zumindest eine erste Spuleneinheit mit einer Erregerspule, einem magnetisierbaren Joch und einer oder mehreren Messspulen auf. Die Stromquelle der Spulenanordnung ist mit der Erregerspule verbunden und eingerichtet, die Erregerspule der zumindest einen Spulenanordnung mit Wechselstrom einer bestimmten Grundfrequenz $f_j$ zu beaufschlagen, die in einem Bereich zwischen 10Hz und 1000Hz liegt. Das bedeutet, dass der Stromquelle eine bestimmte Stromstärke und die Grundfrequenz $f_j$ vorgegeben werden, sodass zu jedem Zeitpunkt die Eregerspulen der Spulenanordnung mit nur einer einzigen Grundfrequenz $f_j$ und der vorgegebenen Stromstärke beaufschlagt werden.

Das magnetisierbare Joch der zumindest einen Spuleneinheit ist erfindungsgemäß U-förmig ausgebildet, einer ersten Ebene $\varepsilon_1$ zugewandt (d.h. die beiden Pole des Jochs sind räumlich näher an der ersten Ebene $\varepsilon_1$ angeordnet als der Rest des Jochs) und wird von der Erregerspule umschlossen. Durch die U-Form des Jochs kann der induzierten magnetischen Feldstärke $H^P$, die in dem Probenmaterial induziert wird, eine Vorzugsrichtung aufgeprägt werden, sodass auch eine korrespondierende Messgröße (die aus den höherharmonischen Anteilen der induzierten magnetischen Feldstärke $H^P$ abgeleitet wird) in vorteilhafter Weise richtungsabhängig in Bezug auf das Probenmaterial ermittelt werden kann.

Weiterhin ist die Steuer- und Messeinheit der erfinsdungsgemäßen Vorrichtung eingerichtet, in zyklischen Messdurchläufen $M_k$ sequentiell eine oder mehrere Grundfrequenzen $f_j$ für die Stromquelle vorzugeben und in den Messspulen ein zu der jeweiligen Grundfrequenz $f_j$ korrespondierendes Antwortsignal $r_j$ zu detektieren. Das bedeutet, dass in jedem einzelnen Messdurchlauf $M_k$ (der Index k bezeichnet hierbei einen bestimmten einzelnen Messdurchlauf von mehreren Messdurchläufen) die Erregerspulen sequentiell (im Sinne von zeitlich aufeinanderfolgend) mit Wechselstrom einer oder mehrerer Grundfrequenzen $f_j$ beaufschlagt werden.

In der Regel ist der Wert der magnetischen Permeabilität $\mu^P$ eines zu vermessenden Flachguts nicht exakt bekannt. Gemäß einer bevorzugten Ausgestaltung der Erfindung besteht daher das magnetisierbare Joch aus einem Material, dessen magnetische Permeabilität $\mu^J$ mindestens 3-fach, bevorzugt mindestens 5-fach, besonders bevorzugt mindestens 10-fach größer als eine (ggf. maximal anzunehmende) magnetische Permeabilität $\mu^P$ des Flachprodukts ist. Zusätzlich oder alternativ dazu ist eine Querschnittsfläche $A^J$ des Jochs mindestens 5-fach, bevorzugt mindestens 10-fach größer als eine effektive Querschnittsfläche $A^P$ des Flachprodukts. Dabei ist, wie voranstehend erwähnt, die Ermittlung einer effektiven Querschnittsfläche $A^P$, durch die der Großteil - beispielsweise mindestens 90% oder 95% - des magnetischen Flusses $\Phi$ innerhalb des Probenmaterials hindurchtritt, dem Fachmann bekannt.

Durch diese Maßnahmen kann bei Anliegen eines magnetischen Wechselfeldes mit einer Grundfrequenz $f_j$ zwischen Joch und Flachgut entsprechend Formel (8) das Verhältnis der magnetischen Feldstärke $H^P$ im Flachgut zur magnetischen Feldstärke $H^J$ im Joch vorteilhaft so eingestellt werden, dass - wie im Grundgedanken der Erfindung voranstehend erläutert - die magnetische Flussdichte B im Flachgut einen Wert im nichtlinearen Bereich der Hysteresekurve annimmt, sodass in diesem höherharmonische Anteile zur Grundfrequenz $f_j$ entstehen, während gleichzeitig die Werte der magnetischen Flussdichte B im Joch weit unterhalb der Sättigungsflussdichte $B_s$ verbleiben, sodass im Joch selbst keine höherharmonischen Anteile erzeugt werden.

In einer bevorzugten Ausgestaltung der erfindungsgemäßen Vorrichtung weist die Spulenanordnung eine zweite Spuleneinheit auf, die identisch zur ersten Spuleneinheit ausgebildet ist. Die zweite Spuleneinheit weist daher ebenfalls eine Erregerspule, ein magnetisierbares Joch und eine oder mehrere Messspulen auf. Die Stromquelle der Spulenanordnung ist in diesem Fall mit der Erregerspule der ersten und der zweiten Spuleneinheit verbunden und eingerichtet, beide Erregerspulen mit Wechselstrom einer bestimmten Grundfrequenz $f_j$ zu beaufschlagen, die in einem Bereich zwischen 10Hz und 1000Hz liegt. Weiterhin ist die zweite Spuleneinheit spiegelsymmetrisch zur ersten Spuleneinheit in Bezug auf die erste Ebene $\varepsilon_1$ angeordnet. Zudem sind die Erregerspulen der ersten und zweiten Spuleneinheit elektrisch in einer Serienschaltung angeordnet.

Durch diese bevorzugte Ausgestaltung kann ein magnetischer Fluss $\Phi$ dem zu vermessenden Flachgut, insbesondere in einem Metallband, das entlang der ersten Ebene $\varepsilon_1$ positioniert wird, vorteilhaft erhöht und noch vollständiger in diesem konzentriert werden. Zudem wird in Verbindung mit der Serienschaltung eine identische Stromstärke in der ersten und zweiten Spuleneinheit erzwungen und eine Phasenverschiebung des Stromes zwischen den beiden Spuleneinheiten vermieden, wodurch vorteilhaft - jeweils eine gleiche Windungsanzahl N und ein baugleiches Joch der beiden Spuleneinheiten vorausgesetzt - der von diesen induzierte magnetische Fluss $\Phi$ räumlich symmetrisch im Probenmaterial ausgeprägt wird. Insgesamt wird durch die zweite, der ersten gegenüberliegende Spulenanordung ein Messfehler aufgrund eines nicht-konstanten Abstandes des Messobjekts zur ersten und zweiten Spuleneinheit während der Messung (z.B. hochfrequente Passlinienschwankung eines Metallbandes an einer Kaltwalzlinie) von etwa 10% (im Falle nur einer Spulenanordnung) auf typischerweise 1% reduziert.

Gemäß einer weiteren bevorzugten Ausgestaltung der erfindungsgemäßen Vorrichtung ist diese eingerichtet, bei zumindest einer bestimmten Grundfrequenz $f_{j0}$ eine bestimmte magnetische Feldstärke $H^{J0}$ zu erzeugen. Die bestimmte Grundfrequenz $f_{j0}$ und die bestimmte magnetische Feldstärke $H^{J0}$ entsprechen dabei einer standardisierten Prüfanordnung. Beispielsweise beträgt die bestimmte magnetische Feldstärke $H^{J0}$ 2500A/m bei einer Grundfrequenz $f_{J0}$ von 50Hz. Durch diese Ausgestaltung können die ermittelten Materialeigenschaften $Q_i$ vorteilhaft besonders genau anhand von Prüfdaten kalibriert werden, die der normierten Prüfanordnung entsprechenden. Aufgrund der zugrundeliegenden Regressionsberechnung überträgt sich die hohe Genauigkeit auch auf jene Materialeigenschaften, die bei einer anderen als der bestimmten Grundfrequenz $f_{J0}$ bzw. einer anderen als der bestimmten magnetischen Feldstärke $H^{J0}$ ermittelt wurden.

Gemäß einer weiteren bevorzugten Ausgestaltung der erfindungsgemäßen Vorrichtung beträgt ein lateraler Abstand $d_l$ zwischen den Polen des Jochs jeder Spuleneinheit mindestens ein Zweifaches, bevorzugt mindestens ein Dreifaches eines charakteristischen Jochdurchmessers $d^J$, wobei der laterale Abstand $d_l$ beispielsweise der Abstand der Mittelpunkte der Polflächen des Jochs ist. Unter dem charakteristischen Jochdurchmesser $d^J$ wird beispielsweise ein Durchmesser bzw. eine größte Kantenlänge eines Querschnitts eines Jochs mit rundem bzw. rechteckigem Querschnitt verstanden.

Durch die genannte Bemaßung wird die Ausbildung einer ausgeprägten Vorzugsrichtung der im Probenmaterial hervorgerufenen magnetischen Feldstärke $H^P$ ermöglicht. Bei kleinerem Verhältnis von $d_l$ zu $d^J$ würde kein größtenteils konstanter räumlicher Verlauf der Feldstärke $H^P$ resultieren und es würde ein nennenswerter Anteil am gesamten, von der Spulenvorrichtung generierten magnetischen Fluss $\Phi$ nicht in das Probenmaterial eintreten, sondern an diesem vorbeilaufen. Weiterhin wird durch den lateralen Abstand $d_l$ das räumliche Auflösungsvermögen der erfindungsgemäßen Vorrichtung vorgegeben.

Gemäß einer weiteren bevorzugten Ausgestaltung umfasst die erfindungsgemäße Vorrichtung weiterhin eine Abstandsmessvorrichtung, die eingerichtet ist, einen ersten Abstand $d_1$ der ersten Spuleneinheit zu einer ersten Oberfläche des Flachprodukts kontinuierlich (z.B. zyklisch mit einer Zykluszeit im Bereich der Dauer eines Messdurchlaufes $M_k$) zu bestimmen. Zudem umfasst die Vorrichtung gemäß dieser Ausgestaltung eine Positioniervorrichtung, die eingerichtet ist, die Spulenanordnung relativ zu der ersten Oberfläche des Flachprodukts zu positionieren, und die Steuer- und Messeinheit ist mit der Positioniervorrichtung steuertechnisch verbunden. Dadurch kann die Steuer- und Messeinheit die Positioniervorrichtung beispielsweise derart ansteuern, dass der erste Abstand $d_1$ einen bestimmten Wert annimmt sowie dass - falls vorhanden - eine zweite Spuleneinheit - relativ zur ersten Spuleneinheit verfahren wird.

Der erste Abstand $d_1$ ist beispielsweise als der Normalabstand zwischen den Polen des Jochs der ersten Spulenanordnung und einer ersten Oberfläche des Flachprodukts, die den Polen zugewandt ist, definiert. Mittels der Astandsmessvorrichtung kann der erste Abstand $d_1$ bestimmt und kontrolliert werden. Weil der induzierte magnetische Fluss $\Phi$ stark von einem Luftspalt zwischen den Polen des Jochs und dem Flachprodukt beeinflusst wird, kann durch eine entsprechende Überwachung bzw. Steuerung des ersten Abstands $d_1$ mit Hilfe der Abstandsmessvorrichtug, der Positioniervorrichtung und der Steuer- und Messeinheit das korrespondierende Messignal in den Messspulen vorteilhaft mit dem ersten Abstand $d_1$ korreliert bzw. konstant gehalten werden.

In einer bevorzugten Ausgestaltung der erfindungsgemäßen Vorrichtung ist die Abstandsmessvorrichtung als eine Anordnung aus einem oder mehreren Lasertriangulationssensoren ausgebildet. Deratige Sensoren sind kostengünstig, können aufgrund ihrer geringe Baugröße in einfacher Weise in ein Schutzgehäuse integriert werden und weisen zudem in dem erfindungsrelevanten Abstandsbereich von 10-100mm eine sehr hohe Messgenauigkeit von typischerweise 100pm oder weniger auf.

Die Vorteile und technischen Wirkungen des erfindungsgemäßen Verfahrens korrespondieren mit jenen der erfindungsgemäßen Vorrichtung. Bei dem erfindungsgemäßen Verfahren mittels einer erfindungsgemäßen Vorrichtung werden eine oder mehrere Materialeigenschaften $Q_i$ des Flachprodukts ermittelt, wobei das Flachprodukt in der ersten Ebene $\varepsilon_1$ der erfindungsgemäßen Vorrichtung platziert oder an der Vorrichtung vorbeibewegt wird. Der Index i steht hierbei für eine bestimmte von mehreren möglichen Materialeigenschaften). 'Platziert' bedeutet in diesem Zusammenhang, dass das Flachprodukt während der Messung relativ zu den Polen fix positioniert ist, wohingegen im Fall von 'vorbeibewegt' eine Bewegung des Flachprodukts relativ zu den Polen gemeint ist, z.B. im Rahmen eines Bandlaufs an einer Kaltwalzanlage.

Die Ermittlung der Materialeigenschaften $Q_i$ erfolgt zyklisch in einem oder mehreren Messdurchläufen $M_k$ (wobei der Indek k einen bestimmten Messdurchlauf bezeichnet). In jedem einzelnen Messdurchlauf $M_k$ werden die Erregerspulen der erfindungsgemäßen Vorrichtung mit Wechselstrom einer oder mehrerer Grundfrequenzen $f_j$ beaufschlagt. Im Falle von mehreren Grundfrequenzen werden dem Wechselstrom diese sequentiell - im Sinne von 'zeitlich aufeinanderfolgend' - aufgeprägt. Alle Grundfrequenzen $f_j$ liegen dabei wiederum in einem Bereich von 10Hz bis 1000Hz.

Durch die Beaufschlagung der Erregerspulen mit Wechselstrom wird in jedem Messdurchlauf $M_k$ in dem Flachprodukt eine entsprechende zeitlich variable magnetische Flussdichte B induziert, die ihrerseits in den Messspulen der erfindungsgemäßen Vorrichtung ein zu der jeweiligen Grundfrequenz $f_j$ korrespondierendes Antwortsignal $r_j$ hervorruft. Dieses Antwortsignal $r_j$ wird erfindungsgemäß - z.B. in Form eines entsprechenden Spannungssignals - detektiert. Aufgrund der spezifischen Materialeigenschaften des Flachprodukts weist das jeweilige Antwortsignal $r_j$ zeitlich gesehen höherharmonische Anteile der Ordnung m in Bezug auf die jeweilige Grundfrequenz $f_j$ auf. Anders ausgedrückt: nichtlineare Rückwirkungen im Material des Flachprodukts erzeugen - aufgrund der darin induzierten hohen magnetischen Flussdichte B, die nahe der Sättigungsflussdichte $B_s$ liegt - im zur Grundfrequenz $f_j$ korrespondierenden Antwortsignal $r_j$ höherharmonische Anteile der Ordnung m, deren jeweilige Frequenz ein m-faches der Grundfrequenz $f_j$ ist. Von den höherharmonischen Anteilen wird eine jeweilige Amplitude $a_j^m$ und eine jeweilige Phasenverschiebung $\varphi_j^m$ einer jeweiligen höherharmonischen Ordnung m in Bezug auf den Wechselstrom der jeweiligen Grundfrequenz $f_j$ ermittelt.

Bevorzugt werden zumindest drei höherharmonische Amplituden $a_j^m$ und/oder drei höherharmonische Phasenverschiebungen $\varphi_j^m$ des zu einer jeweiligen Grundfrequenz $f_j$ korrespondierenden Antwortsignals $r_j$ ermittelt, wobei der Grad einer der höherharmonischen Ordnungen m gleich 1 ist: der Fall m = 1 entspricht dabei jenem Anteil im Antwortsignal $r_j$, der dieselbe Frequenz wie die Grundfrequenz $f_j$ aufweist. Die Ermittlung der Anteile mit m = 1 ist vor allem für die Ermittlung jener Materialeigenschaften $Q_i$ vorteilhaft, deren Einfluss auf das Antwortsignal $r_j$ in erster Linie von der Dämpfung des Flachgutmaterials selbst bestimmt wird (wie z.B. die mittlere Körngröße.

Die Materialeigenschaften $Q_i$ werden anhand einer multiplen linearen Regressionsberechnung, die auf einem Satz von Prüfdaten $T_i$ basiert, unter Verwendung der ermittelten Amplituden $a_j^m$ und Phasenverschiebungen $\varphi_j^m$ ermittelt. Bei dieser Regressionsberechnung werden beispielsweise Regressionskoeffizienten verwendet, die mittels multipler linearer Regressionsanalyse aus dem Satz von Prüfdaten $T_i$ vorab bestimmt worden sind. Dabei umfasst der Satz von Prüfdaten $T_i$ einerseits mittels standardisierter Beprobungsverfahren (z.B. Epsteinrahmen-Test) ermittelte Materialeigenschaften $Q_i$ von einer bestimmten Auswahl an Probestücken. Andererseits umfasst der Satz von Prüfdaten $T_i$ korrespondierend dazu höherharmonische Amplituden $a_j^m$ und Phasenverschiebungen $\varphi_j^m$, die mittels der erfindungsgemäßen Vorrichtung von jener Auswahl an Flachprodukten in der voranstehend beschriebenen Weise ermittelt werden, von denen die Probenstücke extrahiert wurden.

Gemäß einer bevorzugten Ausgestaltung werden die Materialeigenschaften $Q_i$ bei mehreren unterschiedlichen Grundfrequenzen $f_j$ ermittelt, wobei sich alle Grundfrequenzen $f_j$ paarweise voneinander um zumindest den Faktor 2 unterscheiden. Bei einem Messdurchgang wird dabei durch die Erregerspulen Wechselstrom der entsprechenden Grundfrequenzen $f_j$ zeitlich hintereinanderfolgend durchgeleitet. Vorteilhaft ermöglicht eine solche Ermittlung bei mehreren Grundfrequenzen $f_j$ eine genauere Bestimmung der Materialeigenschaften, da die von dem Flachprodukt hervorgerufenen Antwortsignale $r_j$ sowohl von den spezfischen Materialeigenschaften $Q_i$ selbst als auch von der jeweils aufgeprägten Grundfrequenz $f_j$ abhängen. So werden beispielsweise die Antwortsignale $r_j$ in einem Bereich von 10Hz bis 50Hz der Grundfrequenz $f_j$ in erster Linie von den mechanischen Materialeigenschaften materialspezifisch beeinflusst, wobei eine derartige materialspezifische Unterscheidbarkeit Grundvoraussetzung für eine Regressionberechnung bzw. eine Korrelation mit entsprechenden Prüfdaten $T_i$ ist. Im Gegensatz dazu verursachen die elektromagnetischen Materialeigenschaften erst bei höheren Werten der Grundfrequenz $f_j$ eine materialspezifische Auswirkung auf die Antwortsignale $r_j$.

Gemäß einer bevorzugten Ausgestaltung des erfindungsgemäßen Verfahrens umfassen die Materialeigenschaften eine Zugfestigkeit $\sigma_m$ und/oder eine Dehngrenze $R_{p02}$ als mechanische Eigenschaften des Flachprodukts. Alternativ oder zusätzlich werden eine Magnetverlustleistung P und/oder eine magnetische Polarisation J als elektromagnetische Eigenschaften des Flachprodukts ermittelt. Optional können die mechanischen Materialeigenschaften weiterhin eine mittlere Korngröße des Materials des Glachguts umfassen. Die physikalischen Einheiten der Zugfestigkeit $\sigma_m$ und der Dehngrenze $R_{p02}$ werden jeweils in Newton pro Quadratmillimeter [N/mm$^2$] - entsprechend Megapascal [MPa] - angegeben. Die Dehngrenze $R_{p02}$ ist die Zugspannung im einaxialen Zugversuch, bei der eine plastische Extensometer-Dehnung einem Prozentanteil von 0,2% der Extensometer-Messlänge entspricht. Die Magnetverlustleistung P bemisst sich physikalisch in Watt pro kg [W/kg] und wird auf eine bestimmte magnetische Flussdichte B bezogen, die in dem Flachprodukt induziert wird. Die magnetische Polarisation J wird in Tesla [T] angegeben und auf eine bestimmte magnetische Feldstärke H bezogen, die von der erfindungsgemäßen Vorrichtung erzeugt wird.

Beispielsweise werden die Zugfestigkeit $R_m$ und/oder die Dehngrenze $R_{p02}$ bei einer ersten Frequenz $f_1$ ermittelt, die in einem Bereich zwischen 16Hz und 22Hz, bevorzugt zwischen 18Hz und 20Hz liegt. Zusätzlich werden die Magnetverlustleistung P und/oder die magnetische Polarisation J sowohl bei einer zweiten Frequenz $f_2$ als auch bei einer dritten Frequenz $f_3$ ermittelt, wobei die zweite Frequenz $f_2$ in einem Bereich zwischen 45Hz und 55Hz, bevorzugt zwischen 48Hz und 52 Hz, und die dritte Frequenz $f_3$ in einem Bereich zwischen 350Hz und 450Hz, bevorzugt zwischen 380Hz und 420Hz liegt.

Gemäß einer weiteren bevorzugten Ausgestaltung des erfindungsgemäßen Verfahrens erstreckt sich die Vorgabe jeder Grundfrequenz $f_j$ für die Stromquelle in jedem Messdurchlauf $M_k$ über zumindest neun Perioden. Anders ausgedrückt: pro Messdurchlauf $M_k$ und pro vorgegebener Grundfrequenz $f_j$ liegt an der bzw. den Erregerspulen Wechselstrom für die Dauer von zumindest neun Perioden an. Durch diese Auswahl an Perioden wird ein einzelner Messdurchlauf $M_k$ auf eine kurze und dennoch ausreichend lange Zeitspanne begrenzt, innerhalb der eine zuverlässige Auswertung des Antwortsignals $r_j$ (beispielsweise mit Hilfe einer Fast-Fourier-Transformation) möglich ist.

In einer weiteren bevorzugten Ausgestaltung des erfindungsgemäßen Verfahrens umfasst die erfindungsgemäße Vorrichtung eine voranstehend beschriebene Abstandsmessvorrichtung und eine voranstehend beschriebene Positioniervorrichtung. Weiterhin steuert die Steuer- und Messeinheit die Positioniervorrichtung derart an, dass bei jedem Messdurchlauf $M_k$ der erste Abstand $d_1$ einen bestimmten Wert annimmt. Mit Hilfe dieser Ausgestaltung kann vorteilhaft eine Abstandsänderung des zu vermessenden Flachprodukts, beispielsweise eine Drift oder Schwankung der Bandlage eines zu vermessenden kaltgewalzten Metallbandes in einer Kaltwalzstraße, ausgeglichen werden.

In einer weiteren bevorzugten Ausgestaltung des erfindungsgemäßen Verfahrens weist die Spulenanordnung eine voranstehend beschriebene zweite Spuleneinheit auf, die identisch zur ersten Spuleneinheit ausgebildet und spiegelsymmetrisch zur ersten Spuleneinheit in Bezug auf die erste Ebene $\epsilon_1$ angeordnet ist. Zudem sind die Erregerspulen der ersten und zweiten Spuleneinheit - wie voranstehend beschrieben - elektrisch in einer Serienschaltung angeordnet. Weiterhin steuert die Steuer- und Messeinheit die Positioniervorrichtung derart an, dass die zweite Spuleneinheit relativ zur ersten Spuleneinheit derart verfahren wird, dass unter Vorgabe einer Dicke d des Flachprodukts bei jedem Messdurchlauf $M_k$ ein zweiter Abstand $d_2$ der zweiten Spuleneinheit zu einer zweiten Oberfläche des Flachprodukts gleich dem ersten Abstand $d_1$ ist.

Der zweite Abstand $d_2$ ist analog zum ersten Abstand $d_1$ in Bezug auf die zweite Oberfläche des Flachprodukts definiert, wobei die zweite Oberfläche der ersten Oberfläche gegenüberliegt. Die erste und zweite Oberfläche des Flachprodukts sind durch die Dicke d des Flachprodukts entlang von dessen kürzester räumlicher Erstreckung voneinander beabstandet. Bei der genannten Ausgestaltung wird ein rtäumlich symmetrischer magnetischer Fluss $\Phi$ im Probenmaterial - insbesondere in dessen Dickenrichtung - realisiert, was vorteilhaft zu einer vorgenannten Verringerung des Messfehlers beiträgt.

Bei einer weiteren Ausgestaltung des erfindungsgemäßen Verfahrens werden die Materialeigenschaften $Q_i$ entlang zumindest zweier unterschiedlicher Messrichtungen relativ zu einer Referenzrichtung des betreffenden Flachprodukts ermittelt, wobei die Messrichtungen miteinander einen Winkel ungleich 0° einschließen. Dafür kann eine erfindungsgemäße Vorrichtung verwendet werden, deren Spulenanordnung eine erste und eine zweite Spuleneinheit aufweist, und wobei ein lateraler Abstand $d_l$ zwischen den Polen des Jochs der jeweiligen Spuleneinheit in Richtung der ersten bzw. zweiten Messrichtung orientiert ist.

Eine der Messrichtungen kann dabei mit der Referenzrichtung übereinstimmen. Bei der Referenzrichtung kann es sich beispielsweise um eine Walzrichtung eines gewalzten Kaltbandes handeln. In diesem Zusammenhang ist allgemein bekannt, dass durch den Walzvorgang die Kornstruktur des Metalls korrespondierend zur Walzrichtung verändert wird, was sich wiederum auf die Materialeigenschaften auswirkt, die entlang einer bestimmten Richtung relativ zu der Walzrichtung gemessen werden. Dadurch kann vorteilhaft die Richtungsabhängigkeit der ermittelten Materialeigenschaften in Bezug auf die festgelegte Referenzrichtung des Flachprodukts ermittelt werden.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung von Ausführungsbeispielen, die im Zusammenhang mit den Zeichnungen näher erläutert werden. Gleiche Details sind dabei in allen Figuren jeweils mit denselben Bezeichnern gekennzeichnet. Es zeigen:

Figur 1 (FIG 1) einen schematischen Vergleich der Hysteresekurven von Probenmaterial und Jochmaterial,

Figur 2 (FIG 2) eine erste Ausführungsform der erfindungsgemäßen Vorrichtung, und

Figur 3 (FIG 3) eine zweite Ausführungsform der erfindungsgemäßen Vorrichtung.

Figur 1 (FIG 1) zeigt einen schematischen Vergleich der Hysteresekurven eines typischen Probenmaterials (dick durchgezogene Kurve) und jenes Materials, aus dem das magnetisierbare Joch einer ersten oder zweiten Spuleneinheit 41, 42 der erfindungsgemäßen Vorrichtung 1 gefertigt ist (Kurve mit dünnerer Strichbreite). Entlang der horizontalen Achse ist die magnetische Feldstärke H aufgetragen, die an das Probenmaterial bzw. das Joch angelegt wird, während die vertikale Achse die magnetische Flussdichte B bemisst, die daraus bei Anliegen einer periodisch oszillierenden magnetischen Feldstärke H resultiert. Weil derartige Hysteresekurven punktsymmetrisch zum Koordinatenursprung sind, zeigt FIG 1 jeweils nur deren positive Abschnitte.

Die Schnittpunkte der Hysteresekurve des Probenmaterials mit der horizontalen Achse bezeichnen die positive bzw. negative Koerzitivfeldstärke $H_C$ bzw. $-H_C$ des Probenmaterials. In den Punkten $P_1$ und $P_2$ (entsprechend den magnetischen Feldstärken $H_1$ und $H_2$) sind stellvertretend für die magnetische Permeabilität $\mu^P$ des Probenmaterials jeweils eine Tangente (dicke strichlierte Linien) eingezeichnet, die der jeweiligen magnetischen Permeabilität $\mu^P_1$ bzw. $\mu^P_2$ gemäß Formel (4) entsprechen. Die Hysteresekurve des Probenmaterials weist im Punkt $P_1$, d.h. bei der geringeren magnetischen Feldstärke $H_1$, einen noch annähernd linearen Verlauf auf, wohingegen bei der größeren magnetischen Feldstärke $H_2$ im Punkt $P_2$ der Verlauf bereits deutlich nichtlinear ist.

Weiterhin ist in FIG 1 die magnetische Permeabilität $\mu^J$ des Jochmaterials angedeutet, die der Steigung des linearen Abschnitts (wiederum gemäß Formel (4)) der dünneren, durchgezogenen Linie im Bereich um den Koordinatenursprung entspricht und die überall größer als die magnetische Permeabilität des Probenmaterials ist. Der durchgezogen dargestellte Bereich dieser Hysteresekurve repräsentiert jenen Bereich für die magnetische Feldstärke $H^J$ im Joch, die erfindungsgemäß tatsächlich generiert wird, wohingegen der strichliert dargestellte Bereich erfindungsgemäß - wie untenstehend beschrieben - vermieden wird.

Das Jochmaterial weist zudem vernachlässigbare Ummagnetisierungsverluste auf, die bei der Oszillation der angelegten magnetischen Feldstärke $H^J$ entstehen: daraus resultiert für das Jochmaterial eine sehr 'schlanke' Hysteresekurve, sodass diese in FIG 1 als Linie dargestellt ist, die keinen Flächeninhalt umschließt (wobei der Flächeninhalt einer Hysteresekurve proportional zu den Ummagnetisierungsverlusten ist).

Schließlich ist in FIG 1 mittels der horizontalen strichpunktierten Linie die magnetische Sättigungsflussdichte angedeutet; weil diese in der Regel auch für Materialien mit unterschiedlicher magnetischer Permeabilität $\mu$ in derselben Größenordnung liegt, wird die Sättigungsflussdichte sowohl für das Probenmaterial als auch für das Jochmaterial mit demselben Wert - in FIG 1 mit $B_S$ gekennzeichnet - angenommen.

Der voranstehend beschriebene Kerngedanke der Erfindung (dass nämlich bei vergleichbaren Querschnittsflächen, die vom magnetischen Fluss $\Phi$ durchdrungen werden, die magnetische Permeabilität $\mu^J$ des Jochs größer als die magnetische Permeabilität $\mu^P$ des Probenmaterials sein soll) - lässt sich in FIG 1 dadurch ausdrücken, dass ein solches Material für das Joch gewählt wird, dessen Hysteresekurve - zumindest in einem bestimmten Bereich um den Koordinatenursprung - einen 'steileren' Verlauf aufweist als die Hysteresekurve des Probenmaterials (beispielsweise im Punkt $P_3$ bei der magnetischen Feldstärke $H_3$ von Fig 1) .

Dadurch wird entsprechend Formel (10) erreicht, dass die magnetische Feldstärke $H^J$ im Joch entsprechend niedriger ist als die magnetische Feldstärke $H^P$ im Probenmaterial: entsprechend ist auch in FIG 1 mit dem Symbol $H^J$ der Bereich der im Joch erzeugten magnetischen Feldstärke gekennzeichnet (korrespondierend zu dem duchgezogenen Bereich der Hysteresekurve des Jochs), während $\underline{H^P}$ den Bereich der magnetischen Feldstärke, die im Probenmaterial realisiert wird, abdeckt. Anders ausgedrückt: bei geeigneter Wahl des Jochmaterials und entsprechender Bestromung der Erregerspule kann die magnetische Feldstärke $H^P$ im Probenmaterial zu jedem Zeitpunkt derart eingestellt werden, dass sie größer als die magnetische Feldstärke $H^J$ im Joch ist, und zwar derart, dass im Probenmaterial Werte für die magnetische Feldstärke $H^P$ erreicht werden, bei denen die Hysteresekurve des Probenmaterials bereits einen nichtlinearen Verlauf aufweist - dies entspricht beispielsweise dem Punkt $P_2$ in FIG 1. Dadurchwerden die gewünschten höherharmonischen Anteile im Antwortsignal des Probenmaterials erzwungen.

Andererseits wird die korrespondierende Hysteresekurve des Jochmaterials derart durchlaufen, dass die magnetische Feldstärke $H^J$ im Joch - wiederum aufgrund von Formel (10) - zu jedem Zeitpunkt im linearen Bereich der Hysteresekurve des Jochmaterials liegt: anders ausgedrückt wird - bei Anliegen eines oszillierenden magnetischen Wechselfeldes - die in FIG 1 dargestellte Hysteresekurve für das Jochmaterial nicht bis in den nichtlinearen Bereich nahe der Sättigungsflussdichte $B_S$ durchlaufen, sondern nur entlang des durchgezogenen Abschnitts bis zu dem 'Umkehrpunkt' $P_4$ (bzw. dessen nicht dargestelltem, punktgespiegeltem Pendant im negativen Bereich). Weil dieser tatsächlich durchlaufene Bereich somit zur Gänze im linearen Bereich liegt, wird die Entstehung von höherharmonischen Anteilen im Jochmaterial wie vorgesehen verhindert.

Noch kürzer zusammengefasst: durch geeignete Wahl des Jochmaterials und Bestromung der Erregerspule wird erreicht, dass die Hysteresekurve des Probenmaterials bis in den nichtlinearen Bereich durchlaufen wird und so höherharmonische Anteile erzeugt werden, wohingegen gleichzeitig das Durchlaufen der Hysteresekurve des Jochmaterials lediglich innerhalb von deren linearem Bereich erfolgt.

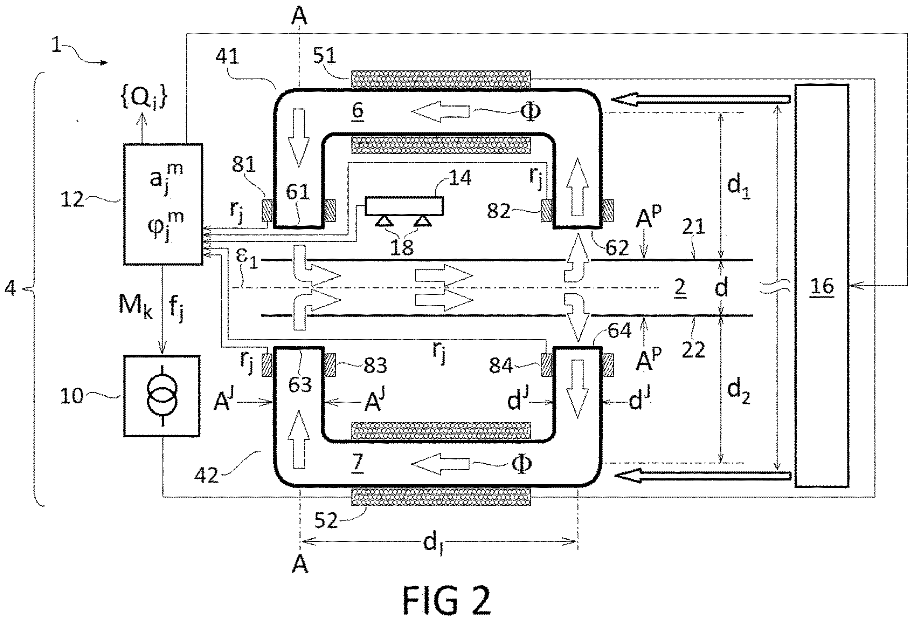

Figur 2 (FIG 2) zeigt eine Ausführungsform der erfindungsgemäßen Vorrichtung 1 mit einer Spulenanordnung 4, die zwei Spuleneinheiten 41 und 42 aufweist. Die erste Spuleneinheit 41 ist in einem ersten Abstand $d_1$ zu einer ersten Oberfläche 21 eines Flachprodukts 2 oberhalb von diesem angeordnet und umfasst eine Erregerspule 51, ein magnetisierbares Joch 6 und zwei Messspulen 81, 82. Das Joch 6 ist U-förmig ausgebildet und weist an seinen beiden Enden in Form von ebenen Polflächen ausgestaltete Pole 61, 62 auf, die einer ersten Ebene $\varepsilon_1$ zugewandt sind, in der auch das zu vermessende Flachprodukt 2 angeordnet ist.

Die Polflächen 61, 62 verlaufen im Wesentlichen parallel zur ersten Ebene $\varepsilon_1$, wobei 'im Wesentlichen' in diesem Zusammenhang bedeutet, dass ein jeweiliger Normalvektor der Polflächen 61, 62 mit dem Normalvektor der ersten Ebene $\varepsilon_1$ einen Winkel von maximal 5°, bevorzugt maximal 2° einschließt. Die Messspulen 81, 82 sind jeweils nahe einer der beiden Polflächen 61, 62 der erste Spuleneinheit 41 angeordnet.

Die zweite Spuleneinheit 42 ist identisch zur ersten Spuleneinheit 41 ausgebildet und spiegelsymmetrisch zu dieser in Bezug auf die erste Ebene $\varepsilon_1$ angeordnet. Die zweite Spuleneinheit 42 umfasst eine Erregerspule 52, ein magnetisierbares Joch 7 mit zwei endseitigen Polflächen 63, 64, die wiederum parallel zur ersten Ebene $\varepsilon_1$ orientiert sind, sowie zwei Messspulen 83, 84. Zudem ist die zweite Spuleneinheit 42 in einem zweiten Abstand $d_2$ zu einer zweiten Oberfläche 22 des Flachprodukts 2 unterhalb von diesem angeordnet. Weiterhin ist im Ausführungsbeispiel in FIG 2 der laterale Abstand $d_l$ als Abtand zwischen den Mittelpunkten der Polflächen 61 und 62 bzw. 63 und 64 des jeweiligen Jochs 6 bzw. 7 definiert.

In dem Ausführungsbeispiel von FIG 2 weisen das Joch 6 und 7 der ersten und zweiten Spuleneinheit einen konstanten Querschnitt entlang ihrer jeweils gerade verlaufenden Abschnitte auf. Der charakteristische Jochdurchmesser $d^J$ und die Querschnittsfläche $A^J$ sind jeweils mittels zweier einander gegenüberliegender, auf das Joch 7 zulaufende Pfeile symbolisiert, die eine Kantenlänge bzw. den Flächeninhalt des quadratischen Querschnitts symbolisieren. Gleichermaßen ist eine effektive Querschnittsfläche $A^P$ des Flachguts 2 mittels zweier einander gegenüberliegender, auf das Flachgut 2 zulaufende Pfeile dargestellt.

In FIG 2 werden der erste bzw. zweite Abstand $d_1$ bzw. $d_2$ in Bezug auf ein Referenzniveau in Höhe der horizontal verlaufenden Abschnitte des jeweiligen Jochs 6, 7 definiert (entsprechend den beiden horizontalen strichpunktierten Referenzlinien); dieses Referenzniveau kann jedoch auch in einer beliebigen anderen Höhe festgelegt werden, beispielsweise in Höhe der Polflächen 61, 62 bzw. 63, 64 der ersten bzw. zweiten Spuleneinheit, weil voraussetzungsgemäß eine Lasertriangulationssensoren 18 umfassende Abstandsmessvorrichtung 14 starr mit der ersten Spuleneinheit 41 (wie in FIG 2 dargestellt) oder mit der zweiten Spuleneinheit 42 verbunden ist. Mittels einer Positioniervorrichtung 16, die mit der ersten und der zweiten Spuleneinheit 41, 42 mechanisch verbunden ist (in FIG 2 mittels zweier dicker horizontaler Pfeile symbolisiert) und die eingerichtet ist, die erste und zweite Spuleneinheit 41, 42 relativ zueinander in einer Richtung normal zur ersten Ebene $\varepsilon_1$ zu verfahren (in FIG 2 durch einen vertikalen Pfeil mit einem "≈"-Symbol angedeutet), kann in der Folge der zweite Abstand $d_2$ eindeutig festgelegt werden.

Eine Steuer- und Messeinheit 12 gibt in zyklischen Messdurchläufen $M_k$ sequentiell eine oder mehrere Grundfrequenzen $f_j$ für eine Stromquelle 10 vor (in FIG 2 durch einen dünnen Pfeil symbolisiert), woraufhin diese die Erregerspule 51 und 52 der ersten und zweiten Spuleinheit 41, 42 mit Wechselstrom der jeweiligen Grundfrequenz $f_j$ beaufschlagt. Die Stromquelle 10 und die Erregerspulen 51 und 52 sind elektrisch in Serie geschaltet (dünne Linien).

Weiterhin steuert die Steuer- und Messeinheit 12 auf Basis eines Abstandswertes, der ihr von der Abstandsmessvorrichtung 14 übermittelt wird (entsprechend dem eingehenden Pfeil in FIG 2) die Positioniervorrichtung 16 derart an (durch einen ausgehenden Pfeil symbolisiert), dass der erste Abstand $d_1$ auf einen bestimmten Wert eingestellt wird. Bei Vorhandensein einer zweiten Spuleneinheit 42 und bei Kenntnis der Dicke d des Flachprodukts 2, die der Steuer- und Messeinheit 12 beispielsweise vonseiten einer Anlagenautomatisierung (in FIG 2 nicht dargestellt) bekannt ist, steuert die Steuer- und Messeinheit 12 die Positioniervorrichtung 16 derart an , dass neben dem ersten Abstand $d_1$ auch der zweite Abstand $d_2$ auf einen bestimmten Wert eingestellt wird, insbesondere auf denselben Wert wie $d_1$.

Die Bestromung der identisch ausgebildeten Erregerspulen 51 und 52 mit Wechselstrom einer bestimmten Grundfrequenz $f_j$ verursacht im magnetisierbaren Joch 6, 7 der ersten und zweiten Spuleneinheit 41, 42 jeweils einen magnetischen Fluss $\Phi$, der in FIG 2 durch breite Pfeile symbolisiert ist und vom Flachprodukt 2 geschlossen wird. Die serielle Verschaltung und Wickelrichtung der Erregerspulen 51 und 52 ist dabei derart ausgestaltet, dass der periodisch oszillierende, aus der ersten Spuleneinheit 41 in das Flachprodukt 2 eintretende magnetische Fluss $\Phi$ - sowohl in Bezug auf seine Richtung als auch in Bezug auf seine Amplitude - zu jedem Zeitpunkt gleich dem magnetischen Fluss $\Phi$ aus der zweiten Spuleneinheit 42 ist (entsprechend der gleich großen und gleichgerichteten Pfeile im Flachprodukt 2).

Die Messspulen 81, 82, 83, 84 der ersten und zweiten Spuleneinheit 41, 42 sind mit der Steuer- und Messeinheit 12 verbunden (engehende Pfeile in FIG 2). In den Messspulen 81, 82, 83, 84 wird während eines Messdurchlaufs $M_k$ entsprechend der momentanen Grundfreqenz $f_j$ des Wechselstromes, mit dem die Erregerspulen 51, 52 beaufschlagt werden, ein jeweiliges Antwortsignal $r_j$ induziert, welches von der Steuer- und Messeinheit 12 erfasst wird. Die Steuer- und Messeinheit 12 extrahiert aus den Messsignalen $r_j$ einer bestimmten Grundfrequenz $f_j$ jeweils mehrere Amplituden $a_j{}^m$ und Phasenverschiebungen $\varphi_j{}^m$ (in Bezug auf den zeitlichen Verlauf des Wechselstroms, mit dem die Erregerspulen 51, 52 beaufschlagt werden) mit der höherharmonischen Ordnung m und ermittelt daraus eine oder mehrere Materialeigenschaften $Q_i$ des Flachprodukts mittels einer Regressionsberechnung, die auf einem Satz von Prüfdaten $T_i$ basiert.

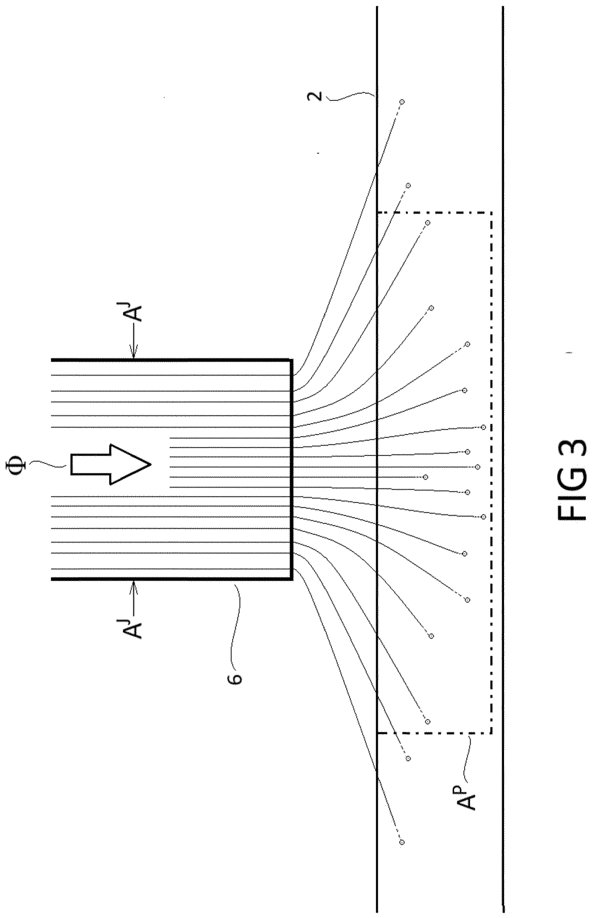

Figur 3 (FIG 3) zeigt eine Detailansicht in Form eines Schnittes durch eine zweite Ausführungsform der erfindungsgemäßen Vorrichtung 1 entlang der strichpunktierten Linien A - A in Fig 2. Die zweiten Ausführungsform umfasst im Gegensatz zur ersten Ausführungsform von FIG 2 nur eine erste Spuleneinheit 41. In dem Joch 6 ist ein momentaner magnetischer Fluss $\Phi$ mittels eines dricken Pfeiles dargestellt; aus dem Joch 6 austretende und in das Flachgut 2 eintretende Linien symbolisieren die magnetischen Feldlinien des magnetischen Flusses $\Phi$, wobei ein strichpunktierter Bereich mit einem kreisförmigen Symbol am Ende jeder Linie die Umlenkung der jeweiligen Feldlinie in dem Flachgut 2 (normal zur Zeichenebene) symbolisiert. Weiterhin ist mittels eines strichpunktierten Rechtecks die effektive Querschnittsfläche $A^P$ innerhalb des Flachguts 2 angedeutet, durch die ein Großteil des magnetische Flusses $\Phi$ innerhalb des Flachguts 2 hindurchtritt und die erfindungsgemäß kleiner als die effektive Querschnittsfläche $A^J$ des Jochs 6 ist. Die Bestimmung der effektiven Querschnittsfläche $A^P$ kann - zumindest näherungsweise - mittels Bestimmung des Verlaufs der magnetischen Feldlinien des magnetischen Flusses $\Phi$ durch Lösung der zugrundeliegenden physikalischen Differentialgleichungen erfolgen, wenn beispielsweise eine untere Grenze der magnetischen Permeabilität $\mu^P$ entsprechend der Qualitätsklasse des zu vermessenden Flachprodukts 2 angenommen wird. Bildlich gesprochen umfasst die effektive Fläche $A^P$ jenen Querschnittsbereich des Flachguts 2, durch den die überwiegende Anzahl der magnetischen Feldlinien hindurchtritt.

Bezugszeichenliste

| 1 | Vorrichtung |
|---|---|
| 2 | Flachprodukt |
| 4 | Spulenanordnung |

| 6, 7 | Joch |
| 10 | Stromquelle |
| 12 | Steuer- und Messeinheit |
| 14 | Abstandsmessvorrichtung |
| 16 | Positioniervorrichtung |
| 18 | Lasertriangulationssensor |
| 21, 22 | erste, zweite Oberfläche |
| 41, 42 | erste, zweite Spuleneinheit |
| 51, 52 | Erregerspule |
| 61, 62, 63, 64 | Pol, Polfläche |
| 81, 82, 83, 84 | Messspule |

| $a_j^m$ | Amplitude |
| $A^J$, $A^P$ | Querschnittsfläche |
| $B$ | magnetische Flussdichte |
| $B_S$ | Sättigungsflussdichte |
| $d$ | Dicke |
| $d^J$ | charakteristischer Jochdurchmesser |
| $d_l$ | lateraler Abstand |
| $d_1$, $d_2$ | erster, zweiter Abstand |
| $f_j$, $f_{j0}$ | Grundfrequenz |
| $H$, $H^{J0}$, $H_1$, $H_2$, $H_3$ | magnetische Feldstärke |
| $\underline{H^J}$, $H^P$ | Bereich magnetische Feldstärke |
| $\overline{H_C}$ | Koerzitivfeldstärke |
| $J$ | magnetische Polarisation |
| $m$ | Grad höherharmonische Ordnung |
| $M_k$ | Messdurchlauf |
| $P$ | Magnetverlustleistung |
| $P_1$, $P_2$, $P_3$, $P_4$ | Punkt in Hysteresekurve |
| $Q_i$ | Materialeigenschaft |
| $r_j$ | Antwortsignal |
| $R_{p02}$ | Dehngrenze |
| $T_i$ | Prüfdaten |
| $\varepsilon_1$ | erste Ebene |
| $\mu^J$, $\mu^P$, $\mu^P_1$, $\mu^P_2$ | magnetische Permeabilität |
| $\varphi_j^m$ | Phasenverschiebung |
| $\sigma_m$ | Zugfestigkeit |
| $\Phi$ | magnetischer Fluss |

## Patentansprüche

**1.** Vorrichtung (1) zum Ermitteln einer oder mehrerer Materialeigenschaften ($Q_i$) eines Flachprodukts (2) mit einer Spulenanordnung (4), einer regelbaren Stromquelle (10) und einer Steuer- und Messeinheit (12),

- wobei die Spulenanordnung (4) zumindest eine erste Spuleneinheit (41) mit einer Erregerspule (51), einem magnetisierbaren Joch (6) und einer oder mehreren Messspulen (81, 82) aufweist,
- wobei die Stromquelle (10) eingerichtet ist, die Erregerspule (51) mit Wechselstrom einer Grundfrequenz ($f_j$) aus einem Bereich von 10Hz bis 1000Hz zu beaufschlagen,
- wobei das Joch (6) der ersten Spuleneinheit (41) U-förmig ausgebildet und einer ersten Ebene ($\varepsilon_1$) zugewandt ist und von der Erregerspule (51) umschlossen wird,
- und wobei die Steuer- und Messeinheit (12) eingerichtet ist, in zyklischen Messdurchläufen ($M_k$) sequentiell eine oder mehrere Grundfrequenzen ($f_j$) für die Stromquelle (10) vorzugeben und in den Messspulen (81, 82) ein zu der jeweiligen Grundfrequenz ($f_j$) korrespondierendes Antwortsignal ($r_j$) zu detektieren.

**2.** Vorrichtung (1) nach Anspruch 1, wobei das Joch (6) aus einem Material besteht, dessen magnetische Permeabilität ($\mu^J$) mindestens 3-fach, bevorzugt mindestens 5-fach, besonders bevorzugt mindestens 10-fach größer als eine magnetische Permeabilität ($\mu^P$) des Flachprodukts (2) ist.

3. Vorrichtung (1) nach Anspruch 1 oder 2, wobei eine Querschnittsfläche ($A^J$) des Jochs (6) mindestens 5-fach, bevorzugt mindestens 10-fach größer als eine effektive Querschnittsfläche ($A^P$) des Flachprodukts (2) ist.

4. Vorrichtung (1) nach einem der vorangegangenen Ansprüche,

   - wobei die Spulenanordnung (4) eine zweite Spuleneinheit (42) aufweist, die identisch zur ersten Spuleneinheit (41) ausgebildet ist und spiegelsymmetrisch zur ersten Spuleneinheit (41) in Bezug auf die erste Ebene ($\varepsilon_1$) angeordnet ist,
   - und wobei die Erregerspulen (51, 52) der ersten und zweiten Spuleneinheit (41, 42) elektrisch in einer Serienschaltung angeordnet sind.

5. Vorrichtung (1) nach einem der vorhergehenden Ansprüche, wobei die Vorrichtung (1) eingerichtet ist, bei zumindest einer bestimmten Grundfrequenz ($f_{j0}$) eine bestimmte magnetische Feldstärke ($H^{J0}$) zu erzeugen, wobei die bestimmte Grundfrequenz ($f_{j0}$) und die bestimmte magnetische Feldstärke ($H^{J0}$) einer standardisierten Prüfanordnung entsprechen.

6. Vorrichtung (1) nach einem der vorhergehenden Ansprüche, wobei ein lateraler Abstand ($d_l$) zwischen den Polen (61, 62, 63, 64) des Jochs (6, 7) jeder Spuleneinheit (41, 42) mindestens ein Zweifaches, bevorzugt mindestens ein Dreifaches eines charakteristischen Jochdurchmessers ($d^J$) beträgt.

7. Vorrichtung (1) nach einem der vorhergehenden Ansprüche,

   - weiterhin umfassend eine Abstandsmessvorrichtung (14), die eingerichtet ist, einen ersten Abstand ($d_1$) der ersten Spuleneinheit (41) zu einer ersten Oberfläche (21) des Flachprodukts (2) kontinuierlich zu bestimmen, und
   - eine Positioniervorrichtung (16), die eingerichtet ist, die Spulenanordnung (4) relativ zu der ersten Oberfläche (21) zu positionieren, und
   - wobei die Steuer- und Messeinheit (12) mit der Positioniervorrichtung (16) steuertechnisch verbunden ist.

8. Vorrichtung (1) nach Anspruch 7, wobei die Abstandsmessvorrichtung (14) als eine Anordnung aus einem oder mehreren Lasertriangulationssensoren (18) ausgebildet ist.

9. Verfahren zum Ermitteln einer oder mehrerer Materialeigenschaften ($Q_i$) des Flachprodukts (2) mittels einer Vorrichtung (1) gemäß einem der Ansprüche 1 bis 8,

   - wobei das Flachprodukt (2) in der ersten Ebene ($\varepsilon_1$) platziert oder an der Vorrichtung (1) vorbeibewegt wird,
   - wobei die Ermittlung zyklisch in einem oder mehreren Messdurchläufen ($M_k$) erfolgt,
   - wobei die Erregerspulen (51, 52) in jedem Messdurchlauf ($M_k$) sequentiell mit Wechselstrom bei einer oder mehreren Grundfrequenzen ($f_j$) in einem Bereich von 10Hz bis 1000Hz beaufschlagt werden,
   - wobei in jedem Messdurchlauf ($M_k$) eine jeweilige Amplitude ($a_j^m$) und eine jeweilige Phasenverschiebung ($\varphi_j^m$) der Ordnung (m) eines in den Messspulen (81, 82, 83, 84) detektierten und zu der jeweiligen Grundfrequenz ($f_j$) korrespondierenden Antwortsignals ($r_j$) ermittelt werden,
   - und wobei die Materialeigenschaften ($Q_i$) anhand einer auf einem Satz von Prüfdaten ($T_i$) basierenden multiplen linearen Regressionsberechnung unter Verwendung der Amplituden ($a_j^m$) und Phasenverschiebungen ($\varphi_j^m$) ermittelt werden.

10. Verfahren nach Anspruch 9, wobei zumindest drei höherharmonische Amplituden ($a_j^m$) und/oder Phasenverschiebungen ($\varphi_j^m$) der jeweiligen höherharmonischen Ordnung (m) des zu einer jeweiligen Grundfrequenz ($f_j$) korrespondierenden Antwortsignals ($r_j$) ermittelt werden, wobei der Grad einer der höherharmonischen Ordnungen (m) gleich 1 ist.

11. Verfahren nach Anspruch 9 oder 10, wobei die Materialeigenschaften ($Q_i$) bei jeweils mehreren Grundfrequenzen ($f_j$) ermittelt werden und wobei sich alle Grundfrequenzen ($f_j$) paarweise voneinander um zumindest den Faktor 2 unterscheiden.

12. Verfahren nach einem der Ansprüche 9 bis 11, wobei die Materialeigenschaften ($Q_i$) eine Zugfestigkeit ($\sigma_m$) und/oder eine Dehngrenze ($R_{p02}$) und/oder eine Magnetverlustleistung (P) und/oder eine magnetische Polarisation (J) umfassen.

**13.** Verfahren nach einem der Ansprüche 9 bis 12, wobei sich die Vorgabe jeder Grundfrequenz ($f_j$) für die Stromquelle (10) in jedem Messdurchlauf ($M_k$) über zumindest neun Perioden erstreckt.

**14.** Verfahren nach einem der Ansprüche 9 bis 13, wobei die Vorrichtung (1) weiterhin

- eine Abstandsmessvorrichtung (14), die eingerichtet ist, einen ersten Abstand ($d_1$) der ersten Spuleneinheit (41) zu einer ersten Oberfläche (21) des Flachprodukts (2) kontinuierlich zu bestimmen, und
- eine Positioniervorrichtung (16), die eingerichtet ist, die Spulenanordnung (4) relativ zur ersten Oberfläche (21) zu positionieren,

umfasst, und wobei

- die Steuer- und Messeinheit (12) die Positioniervorrichtung (16) derart ansteuert, dass bei jedem Messdurchlauf ($M_k$) der erste Abstand ($d_1$) einen bestimmten Wert annimmt.

**15.** Verfahren nach Anspruch 14, wobei

- wobei die Spulenanordnung (4) eine zweite Spuleneinheit (42) aufweist, die identisch zur ersten Spuleneinheit (41) ausgebildet und spiegelsymmetrisch zur ersten Spuleneinheit (41) in Bezug auf die erste Ebene ($\varepsilon_1$) angeordnet ist,
- wobei die Erregerspulen (51, 52) der ersten und zweiten Spuleneinheit (41, 42) elektrisch in einer Serienschaltung angeordnet sind, und
- wobei die Steuer- und Messeinheit (12) die Positioniervorrichtung (16) derart ansteuert, dass die zweite Spuleneinheit (42) relativ zur ersten Spuleneinheit (41) derart verfahren wird, dass unter Vorgabe einer Dicke (d) des Flachprodukts (2) bei jedem Messdurchlauf ($M_k$) ein zweiter Abstand ($d_2$) der zweiten Spuleneinheit (42) zu einer zweiten Oberfläche (22) des Flachprodukts (2) gleich dem ersten Abstand ($d_1$) ist.

FIG 1

EP 4 764 486 A1

FIG 2

EP 4 764 486 A1

FIG 3

Europäisches
Patentamt

European
Patent Office

Office européen
des brevets

# EUROPÄISCHER RECHERCHENBERICHT

Nummer der Anmeldung

EP 24 22 0940

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | JP 4 512079 B2 (NIPPON STEEL CORP) 28. Juli 2010 (2010-07-28) * Absätze [0017], [0023] - [0024], [0039] - [0041]; Abbildung 1 * ----- | 1,3-6,9, 12,15 | INV. G01N27/90 |
| X | EP 1 629 273 B1 (GEN ELECTRIC [US]) 19. März 2008 (2008-03-19) * Absatz [0025]; Abbildungen 4,12 * ----- | 1,5,9-13 | |
| X | JP 2012 184931 A (KOBE STEEL LTD) 27. September 2012 (2012-09-27) * Absätze [0001], [0019], [0022], [0024], [0027], [0031]; Abbildung 1 * ----- | 1,7-9, 12,14 | |
| X | US 2023/018264 A1 (THALE WERNER [DE] ET AL) 19. Januar 2023 (2023-01-19) * Absätze [0002], [0021], [0042]; Abbildung 2 * ----- | 1,2,9 | |

RECHERCHIERTE SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| München | 14. Mai 2025 | De Masi, Rita |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 24 22 0940

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-05-2025

| Im Recherchenbericht angeführtes Patentdokument | | Datum der Veröffentlichung | Mitglied(er) der Patentfamilie | | Datum der Veröffentlichung |
|---|---|---|---|---|---|
| JP 4512079 | B2 | 28-07-2010 | JP | 4512079 B2 | 28-07-2010 |
|  |  |  | JP | 2008122138 A | 29-05-2008 |
| EP 1629273 | B1 | 19-03-2008 | AU | 2003233645 A1 | 21-01-2005 |
|  |  |  | CN | 1771436 A | 10-05-2006 |
|  |  |  | DE | 60319885 T2 | 27-11-2008 |
|  |  |  | EP | 1629273 A1 | 01-03-2006 |
|  |  |  | WO | 2004106912 A1 | 09-12-2004 |
| JP 2012184931 | A | 27-09-2012 | KEINE | | |
| US 2023018264 | A1 | 19-01-2023 | CA | 3161473 A1 | 17-06-2021 |
|  |  |  | DE | 102019133799 A1 | 10-06-2021 |
|  |  |  | EP | 4073502 A1 | 19-10-2022 |
|  |  |  | ES | 2995234 T3 | 07-02-2025 |
|  |  |  | PL | 4073502 T3 | 20-01-2025 |
|  |  |  | US | 2023018264 A1 | 19-01-2023 |
|  |  |  | WO | 2021116109 A1 | 17-06-2021 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102019109337 B3
- EP 0833150 B1
- US 5144565 A

**In der Beschreibung aufgeführte Nicht-Patentliteratur**

- Online Quality Monitoring of IF and High Strength Steels on Continuous Galvanizing Lines Controlled by Furnace Mathematical Model. *Messe METEC & 2nd ESTAD*, 2015
- *Materialcharakterisierung an Flachprodukten verbessert die Prozesskontrolle*, 2012, vol. 07, 80-90